# EUROPEAN PATENT APPLICATION

(11) **EP 1 547 604 A1**
(43) Date of publication of application: **29.06.2005**
(21) Application number: 03791378.7
(22) Date of filing: 28.08.2003
(51) Int. Cl.: A61K 31/663, A61K 9/06, A61K 47/34, A61K 47/36, A61K 47/46, A61P 1/02, A61P 19/08, A61P 43/00

(54) **MEDICINAL COMPOSITION FOR PERIODONTAL POCKET ADMINISTRATION CONTAINING BISPHOSPHONIC ACID DERIVATIVE OR ITS SALT AS THE ACTIVE INGREDIENT**

(30) Priority: 29.08.2002 JP 2002249838
(71) Applicant: TORAY INDUSTRIES, INC., Tokyo 103-8666 (JP)
(72) Inventor: YOSHII, Ryoji, Kamakura-shi, Kanagawa 248-0036 (JP); AOKI, Takao, Ninomiya-cho, Naka-gun, Kanagawa 259-013 (JP)
(74) Representative: Hofer, Dorothea, Dipl.-Phys.
(86) International application number: PCT/JP2003/010940
(87) International publication number: WO 2004/019957

(57) **Abstract**

A pharmaceutical composition for being administered to periodontal pockets, comprising as an effective ingredient a bisphosphonic acid derivative, which has an excellent sustained-release property when locally administered, is disclosed. The pharmaceutical composition for being administered to periodontal pockets according to the present invention comprises a bisphosphonic acid derivative or a salt thereof, and a base which undergoes liquid-gel phase transition upon contacting with physiological body fluid in the periodontal pocket.

## Description

### Technical Field

The present invention relates to a pharmaceutical composition containing a bisphosphonic acid or a derivative thereof, which is excellent for local administration. The pharmaceutical composition for being administered to periodontal pockets according to the present invention is suitably applied to therapies of periodontal diseases.

### Background Art

Periodontal diseases are caused by progress of chronic gingivitis to the state wherein inflammation extends to periodontal tissues other than gingiva, and accompanies progressive destruction of periodontal tissues. Clinically, chronic inflammation of gingiva, bleeding from periodontal pockets and involution of alveolar bone are observed, and it is known that teeth are loosen or moved due to the progress of the destruction, and eventually, the teeth naturally drop or are necessitated to be extracted. Therapies of periodontal diseases currently conducted include removal of the causative plaques and dental calculi, root plaining aiming at reattachment of gingiva and removal of necrosed tissues by periodontal surgery (Periodontology (second edition), pp.215-226 (1992) published by Ishiyaku Publishers, Inc). For chemotherapies against periodontal diseases, tetracycline antibiotics are mainly used. However, at present, no drugs exist which directly act on the involution of alveolar bone, which is a critical picture. A drug having such an action is expected to be useful as a new therapeutic drug against periodontal diseases.

Bisphosphonic acid is a structural analogue of pyrophosphoric acid, which is stable in the body, and has biological actions such as ectopic calcification-inhibiting action and bone resorption-inhibiting action. Bisphosphonic acid has already been clinically used as a therapeutic drug for tumor-induced hypercalcemia, Paget's disease of bone, osteoporosis, rheumatoid arthritis and the like, and action against resorption of alveolar bone by periodontal diseases is also expected. Inhibiting action against destruction of periodontal tissues by bisphosphonic acid in rats has been reported (J. Dent. Res. 11, 1430-1433 (1988)). It was also reported recently that alendronate which is a second generation bisphosphonic acid inhibited destruction of alveolar bone in simian and canine periodontal disease models by oral administration (J. Periodontol 63, 825-830 (1992);J. Periodontol 66 (3), 211-217 (1995)).

Incadronate disodium is a third generation bisphosphonic acid having a strong bone resorption-inhibiting action (Japanese Patent Publication (Kokoku) No. 7-629). It has been reported that incadronate disodium exhibits inhibiting action against involution of alveolar bone in hamster periodontal disease models (Journal of Oral Biosciences 36 (5), 510-519 (1994)), and exhibits action against involution of alveolar bone and action of improvement in attachment level in canine periodontal disease models by subcutaneous or oral administration (J. Periodont. Res. 33, 196-204 (1998)).

It is thought that in case of systemic administration such as oral administration, subcutaneous injection or intravenous injection, bisphosphonic acid at a concentration by which the pharmaceutical effect is expressed reaches the affected area at the alveolar bone. However, since the bisphosphonic acid at that concentration also reaches to other tissues so that it may exhibit bone resorption-inhibition action similarly in the bone tissues of the whole body, which may bring about an undesirable effect. In view of this, it has been tried to locally administer bisphosphonic acid. For example, it has been reported that local administration of risedronate to the vicinity of first molar, and local submucosal administration of pamidronate to the palate are effective in rat models of experimental movement of molars (J. Dent. Res. 73 (8), 1478-1484 (1994) ; Orthod. Waves 57 (5), 307-317 (1998)).

However, to date, an agent for inhibiting involution of alveolar bone by local administration of bisphosphonic acid, which may be clinically applied to human, has not been reported at all. Japanese Laid-open PCT Application (Kohyo) No. 7-502506 discloses a therapeutic composition against periodontal diseases, which contains alendronate which is a second generation bisphosphonic acid. However, the main administration route thereof is systemic administration such as oral administration or intravenous injection. As for the local administration the composition, although it is described that the composition may be directly applied to the teeth and inflammatory site of gingiva, the dose and the site of administration are not concretely disclosed, so that it discloses nothing more than a mere possibility. Further, since the teeth and the inflammatory site in gingiva arc always washed by saliva and exudate from gingival sulcus, with the method of directly applying the composition, the drug is readily washed away, so that reaching a high level of alendronate to the alveolar bone is not expected at all.

Under these circumstances, Japanese Laid-open Patent Application (Kokai) Nos. 2001-213779 and 2001-213780 disclose pharmaceutical compositions for injection by which bisphosphonic acid is locally administered by implantation into alveolar mucosa. Since this composition is directly injected into alvcolar mucosa, a high level of drug may be delivered to the alveolar bone. However, this method requires technical burden for physicians and physical burden of patients accompanied by the implantation.

Japanese Laid-open PCT Application (Kohyo) No. 2000-504718 discloses a formulation for local administration comprising bisphosphonic acid in gelatin as a sustained-release base. Since this formulation is administered by embedding a solid formulation in the vicinity of the alveolar bone after periodontal surgery, this formulation requires more technical burden for physicians and more physical burden of patients than the above-mentioned method for implanting bisphosphonic acid into alveolar mucosa.

On the other hand, as the formulations for therapies of local diseased areas in oral cavity, liquid formulations and ointments have already been used. Although liquid formulations can deliver the drugs to all parts in the oral cavity, since they are readily flown away together with saliva, it is difficult to deliver the drugs to alveolar bone. With ointments, although the degree of washing away by saliva is smaller when compared with liquid formulations, the effect is not sufficient, and it cannot be expected to deliver a high level of drug to the alveolar bone by such a formulation. Various types of formulations by which the degree of washing away by saliva is reduced, and by which the drug is sustainedly released in the oral cavity have been proposed. As the sustained-release formulations of oral cavity muco-adhering type, which are attached to the oral mucosa and sustainedly release the drugs, tablets, film formulations and the like have been proposed. However, although these formulations are administered by being attached to the oral mucosa or the outer side of gingiva (gingival epithelium opposite to the root of teeth), since the vascular system in the gingiva is well developed, the drugs ride on the blood flow in the vessels and moves to the whole body, so that it is difficult to selectively deliver the drugs to alveolar bone. In addition, when the formulation is attached to the outer side of the gingiva, since the contact with saliva is frequent, erosion of the formulation is unavoidable, so that the sustained-release of the drugs is limited.

Japanese Patent No. 3051154 discloses a solid formulation for being inserted into periodontal pockets, comprising a biodegradable polymer (a copolymer of lactide and glycolide) containing a drug. Japanese Laid-open Patent Application (Kokai) No. 2001-163768 and Japanese Laid-open PCT Application (Kohyo) No. 2001-504439 disclose sustained-release solid formulations using the muco-adhesive base (in the forms of stick, film and strip) for being inserted into interdentium or periodontal pockets. With these formulations, movement of the drug into the vascular system, which is problematic when the formulation is administered to the outer side of the gingiva, can be avoided. Further, since the formulations are slowly dissolved or hydrolyzed in the periodontal pockets, sustained-release of the drug for a long time may be attained. However, there is a problem in that since these formulations are highly adhesive to the mucosa, they may be attached to other portions before being inserted into periodontal pockets, and at that time, they may be softened by saliva so that the insertion into the periodontal pockets may become difficult. Further, the depth of periodontal pockets in the patients suffering from severe periodontal diseases may be as long as 10 mm or more, and it has been pointed out that administration of these solid formulations into the deep area of such periodontal pockets is very troublesome to the physicians and technically very difficult. Still further, although these publications enumerate a number of drugs such as antibacterial agents and antibiotics, they are totally silent about bisphosphonic acid.

From these view points, the characteristics of the pharmaceutical composition for therapies of periodontal diseases, which are required for the selective delivery of the drug to the alveolar bone, are that the composition may be administered to periodontal pockets, the composition may be easily administered, the composition has a fluidity or viscosity that enables the composition to be administered into the deep portion of periodontal pockets, and that the composition gives sustained-release of the drug for a long time *in situ* after being administered into the periodontal pockets.

As a formulation which has a fluidity before being administered to periodontal pockets, and which gives sustained-release of the drug at the site of administration, Japanese Patent Publication (Kokoku) No. 2-34325 discloses a therapeutic composition against periodontal diseases, comprising minocycline, a magnesium compound, a water-soluble macromolecular compound, a polyol, ethyl methacrylate/trimethylammonium ethyl chloride methacrylate copolymer and a solubilizer. It is disclosed that since this composition is in the form of ointment and has adhesiveness, when the composition is administered to periodontal pockets, the composition is retained therein, and that the plasticizer in the composition is replaced with saliva so that a film of the copolymer is formed, and minocycline is sustainedly released through the film. On the other hand, as another dosage form, Japanese Patent Publication (Kokoku) No. 6-67853 discloses an ophthalmic composition containing a polysaccharide which gels upon contact with lacrimal fluid. This ophthalmic composition is administered to eyes in the form of liquid, and gels *in situ* by the effect of the lacrimal fluid to increase the ion strength, thereby promoting the sustainment of the drug release. However, this publication is totally silent about bisphosphonic acid and therapies of periodontal diseases, and is also silent about the administration to periodontal pockets.

One reason why the administration of bisphosphonic acid into periodontal pockets has not been studied at all is that bisphosphonic acid forms a hardly absorbable insoluble salt in the presence of calcium ion. This is well-known by those skilled in the art, and the fact that calcium ion abundantly exist in saliva is also well-known. Thus, since administration of bisphosphonic acid to periodontal pockets, *inter alia*, administration of bisphosphonic acid contained in a formulation which exhibits sustained-releasing function upon contact with saliva, has a concern about formation of the insoluble salt due to the contact of the bisphosphonic acid and the calcium ion in the saliva for a long time, such administration has not been studied by those skilled in the art.

As described above, nobody has studied a pharmaceutical composition for therapy of periodontal diseases comprising bisphosphonic acid as an effective ingredient, which does not impose burdens to physicians and patients by the necessity of injection or surgery, which may be administered to periodontal pockets, which may be administered simply, which has a fluidity or viscosity that enables the composition to be administered into the deep portion of periodontal pockets, which does not form the insoluble salt of bisphosphonic acid by calcium ion, and which sustainedly release bisphosphonic acid in the periodontal pockets.

### Disclosure of the Invention

An object of the present invention is to provide a pharmaceutical composition for being administered to periodontal pockets, comprising a bisphosphonic acid derivative, which has excellent sustainment of drug release when locally administered.

The present inventors intensively studied to discover that by using a base which undergoes liquid-gel phase transition upon contact with physiological body fluid in the periodontal pocket as the base for the administration of the bisphosphonic acid derivative, sustained-release of the bisphosphonic acid derivative in the periodontal pocket may be attained, which bisphosphonic acid derivative was hitherto thought difficult to be administered to periodontal pockets, and the bisphosphonic acid derivative may be selectively delivered to the alveolar bone, thereby completing the present invention.

That is, the present invention provides a pharmaceutical composition for being administered to periodontal pockets, comprising a bisphosphonic acid derivative or a salt thereof, and a base which undergoes liquid-gel phase transition upon contact with physiological body fluid in the periodontal pocket.

The pharmaceutical composition for being administered to periodontal pockets, comprising a bisphosphonic acid derivative or a salt thereof, is administered in the ungelled form, and the composition gels at the administered site. By this, the sustained-release of the bisphosphonic acid compounds in the periodontal pockets may be attained, which bisphosphonic acid compounds were hitherto thought difficult to be administered to periodontal pockets, and the bisphosphonic compounds may be selectively delivered to the alveolar bone, so that the composition shows an excellent effect as a alveolar bone resorption-inhibiting agent and as a therapeutic composition against periodontal diseases.

### Brief Description of the Drawing

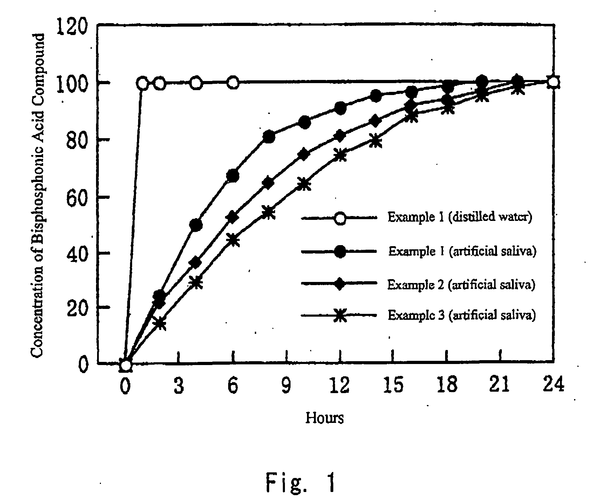

Fig. 1 shows the change of the concentration of bisphosphonic acid derivative in each test fluid (distilled water or artificial saliva) with time after immersing each of the compositions prepared in Examples of the present invention in the test fluid. Concentration of the bisphosphonic acid compound in the test fluid is taken along the ordinate, and the concentration is expressed in terms of percentage taking the concentration attained when the entire bisphosphonic compound in each composition is eluted as 100. The hours passed after immersion of each composition in the test fluid is taken along the abscissa.

### Best Mode for Carrying Out the Invention

As the bisphosphonic acid derivative or salt thereof used in the present invention, compounds and salts thereof, which arc structural analogues of pyrophosphoric acid, which are stable in the body, which have two phosphonic acid residues, and which have bone resorption-inhibiting actions may preferably be used. Preferred examples thereof include 4-amino-1-hydroxybutylidene-1,1-bisphosphoinc acid: alendronate, N,N-dimethyl-3-amino-1-hydroxypropylidene-1,1-bisphosphonic acid: mildronatc, olpadronate, 1-hydroxy-3-(N-methyl-N-pentylamino)propylidene-1,1-bisphosphonic acid: ibandronate, 1-hydroxy-2-(3-pyridyl)ethylidene-1,1-bisphosphonic acid: risedronate, 1-hydroxyethylidene-1,1-bisphosphonic acid: ethidronate, 1-hydroxy-3-(1-pyrrolidinyl)propylidene-1,1-bisphosphonic acid, 1-hydroxy-2-(1-imidazolyl)ethylidene-1,1-bisphosphonic acid: zoledronate, 1-hydroxy-2-(imidazo[1,2-a]pyridine-3-yl)ethylidene-1,1-bisphosphonic acid: minodronate, 1-(4-chlorophenylthio)methylidene-1,1-bisphosphonic acid: tildronate, 1-(cycloheptylamino)methylidene-1,1-bisphosphonic acid: cimadronate, incadronate, 6-amino-1-hydroxyhexylidene-1,1-bisphosphonic acid: neridronate, I-hydroxy-2-(imidazo[1,2-a]pyridine-3-yl)ethane-1,1-bisphosphonic acid, and methane bisphosphonic acid derivatives represented by Formula (I): 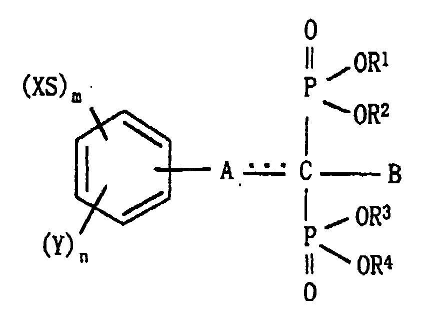 [wherein X represents C₁-C₈ linear or branched alkyl or cycloalkyl (in case of cycloalkyl, the number of carbon atoms is 3 to 8) which is not substituted or which has (a) substituent(s) having nitrogen, oxygen and/or silicon atom(s), phenyl or naphthyl (the phenyl or naphthyl may be substituted by C₁-C₈ linear or branched alkyl or cycloalkyl (in case of cycloalkyl, the number of carbon atoms is 3 to 8), C₁-C₈ linear or branched alkoxy, halogen and/or hydroxy); Y represents C₁-C₈ linear or branched alkyl, trifluoromethyl, C₂-C₈ linear or branched alkenyl, C₃-C₈ cycloalkyl, C₁-C₈ alkoxy or halogen (excluding chlorine substituting atp-position); m and n independently represent 0, 1, 2 or 3; · · · represents double bond or single bond; A represents -(D)_{b}-(CH₂)_{c}- (wherein D represents sulfur, oxygen, NR⁵ (wherein R⁵ represents hydrogen or C₁-C₈ linear or branched alkyl or cycloalkyl (in case of cycloalkyl, the number of carbon atoms is 3 to 8), D binding directly to the methane bisphosphonic acid, c represents an integer of 0 to 3, b represents 0 or 1), or -(CH=CH)_{d}-CH= (wherein d represents 0 or 1, and when A is -(CH=CH)_{d}-CH=, B does not exist); B represents hydrogen, C₁-C₈ linear or branched alkyl or cycloalkyl (in case of cycloalkyl, the number of carbon atoms is 3 to 8), hydroxy or trialkylsiloxy (each of the alkyl groups therein is C₁-C₈ linear or branched alkyl or cycloalkyl (in case of cycloalkyl, the number of carbon atoms is 3 to 8); R¹, R², R³ and R⁴ represent, the same or different, hydrogen, C₁-C₈ linear or branched alkyl or cycloalkyl (in case of cycloalkyl, the number of carbon atoms is 3 to 8), or a pharmaceutically acceptable cation].

In the above-described Formula (I), "(XS)ₘ-" means that the substituent represented by XS in the number of m is(are) bound to the benzene ring. In cases where m is 2 or 3, the substituents represented by XS may be the same or different. Similarly, in Formula (I), "(Y)ₙ-" means that the substituent represented by Y in the number of n is(are) bound to the benzene ring. In cases where n is 2 or 3, the substituents represented by Y may be the same or different. As described above, n and m independently represent 0, 1, 2 or 3. However, as evident from the chemical structure of Formula (I), the maximum of m + n is necessarily 5.

Among the bisphosphonic acid derivatives described above, those represented by Formula (I) are preferably employed.

Among the methane bisphosphonic acid derivatives represented by Formula (I), those having the following substituents are preferred: Preferred examples of the C₁-C₈ linear or branched alkyl or cycloalkyl (in case of cycloalkyl, the number of carbon atoms is 3 to 8) which is not substituted or which has (a) substituent(s) having nitrogen, oxygen and/or silicon atom(s) as the substituent X include methyl, ethyl, propyl, isopropyl, butyl, isobutyl, t-butyl, pentyl, hexyl, cyclobutyl, cyclopcntyl, cyclohcxyl, cyclopentylmethyl, cyclohexylmethyl, 2-aminoethyl, 2-N-methylaminoethyl, 2-N,N-dimethylaminoethyl, 2-hydroxyethyl, 2-alkoxyethyl, 2-trialkylsiloxyethyl, 2-aminopropyl, 2-N-methylaminopropyl, 2-N,N-dimethylaminopropyl, 3-aminopropyl, 3-N-methylaminopropyl, 3-N,N-dimethylaminopropyl, 2-hydroxypropyl, 2-alkoxypropyl, 2-trialkylsiloxypropyl and the like. As is apparent from these examples, the cycloalkyl group may be branched. Other Xs are phenyl, substituted phenyl, naphthyl and substituted naphthyl. Examples of the C₁-C₈ linear or branched alkyl or cycloalkyl (in case of cycloalkyl, the number of carbon atoms is 3 to 8) which is the substituent of the phenyl or naphthyl group include methyl, ethyl, propyl, isopropyl, butyl, isobutyl, t-butyl, pentyl, hexyl, cyclopentylmethyl, cyclohexylmethyl and the like. As is apparent from these examples, the cycloalkyl group may be branched. Examples of the C₁-C₈ linear or branched alkoxy include methoxy, ethoxy, n-propoxy, isopropoxy, n-butoxy, pentoxy, hexoxy and the like. The halogen may be fluorine, chlorine, bromine or iodine. The position(s) of the substituent(s) XS is(are) ortho-, meta- and/or para- with respect to A.

Examples of the C₁-C₈ linear or branched alkyl as the substituent Y include methyl, ethyl, propyl, isopropyl, butyl, isobutyl, t-butyl, pentyl, hexyl and the like. Examples of the C₂-C₈ linear or branched alkenyl include vinyl, allyl, 1-propenyl, isopropenyl, butenyl, pentenyl and the like. Examples of the C₃-C₈ cycloalkyl include cyclopropyl, cyclobutyl, cyclopentyl, cyclohexyl, cylopentylmcthyl, cyclohexylmethyl and the like. As is apparent from these examples, the cycloalkyl group may be branched. Examples of the C₁-C₈ alkoxy include methoxy, ethoxy, n-propoxy, isopropoxy, n-butoxy, pentoxy, hexoxy and the like. The halogen may be fluorine, chlorine (excluding the chlorine substituting at the para- position with respect to A), bromine or iodine. The position(s) of the substituent(s) Y is(are) not restricted.

In cases where A represents -(D)_{b}-(CH₂)_{c}- and · · · represents single bond, D is sulfur, oxygen, NR⁵ (wherein R⁵ represents hydrogen or C₁-C₈ linear or branched alkyl) or CH₂, c is 0, 1, 2 or 3, and b is 0 or 1 (with the proviso that when b=0, c=0). More preferably, b and c independently are 0 or 1. In cases where B is hydroxy or trialkylsiloxy (each of the alkyl groups therein is C₁-C₈ linear or branched alkyl), D is sulfur, oxygen or NR⁵ (R⁵ represents the same meanings as described above), and b=1, those wherein c=0 are chemically unstable and so not preferred. Even in these cases, however, those wherein c is 1, 2 or 3 are stable and preferred. Especially preferred examples of A include S, NH, O, CH₂, CH₂CH₂, SCH₂, SCH₂CH₂, SCH₂CH₂CH₂, NHCH₂, OCH₂ and the like. The compounds in which the phenyl group is directly bound to the carbon atom of the methane bisphosphonic acid and not through A (that is, the cases where b=c=0) are also included. The cases where A represents -(CH=CH)_{d}-CH= means the cases where · · · represents double bond and B docs not exist, wherein d is 0 or 1. Examples of the C₁-C₈ linear or branched alkyl or cycloalkyl represented by B, R¹, R², R³, R⁴ or R⁵ include methyl, ethyl, propyl, isopropyl, butyl, isobutyl, t-butyl, pentyl, hexyl, cyclopentylmethyl, cyclohexylmethyl and the like. As is apparent from these examples, the cycloalkyl group may be branched. In cases where B is trialkylsiloxy (each of the alkyl groups therein is C₁-C₈ linear or branched alkyl or cycloalkyl (in case of cycloalkyl, the number of carbon atoms is 3 to 8)), preferred examples of the C₁-C₈ linear or branched alkyl or cycloalkyl arc also the same as those just mentioned above. Examples of the pharmaceutically acceptable cation represented by R¹, R², R³ or R⁴ include metal cations, ammonium NR⁶ (wherein R⁶ represents hydrogen or C₁-C₈ linear or branched alkyl) and the like. Especially preferred metal cations are alkaline metal ions such as lithium, sodium and potassium ions; and alkaline earth metal ions such as magnesium and calcium ions. However, other metal cations such as aluminum, zinc and iron cations arc also within the scope of the present invention. The ammonium include ammonia, primary amines, secondary amines, tertiary amines and quaternary ammonium. Examples of these include ammonium such as ammonia, methylamine, dimethylaminc, trimethylamine, ethylamine, diethylamine, triethylamine, propylamine, dipropylamine, isopropylamine, diisopropylamine, butylamine, dibutylamine, isobutylamine, t-butylamine, monoethanolamine, diethanolamine, triethanolamine, and tetramethylammonium, tetraethylammonium and the like. Among these, cations of sodium, potassium, ammonia and alkylamines are preferred. The cations represented by R¹ to R⁴ may be the same or different. Further, those wherein a part thereof is(are) cations and other part thereof is(are) hydrogen, e.g., monocationic salt, dicationic salt and tricationic salt are also within the scope of the present invention. Preferred methane bisphosphonic acid derivatives represented by Formula (I) are those wherein all of R¹ to R⁴ are hydrogen, three of R¹ to R⁴ are hydrogen and the remaining one is sodium, three of R¹ to R⁴ are hydrogen and the remaining one is ammonium, two of R¹ to R⁴ are hydrogen and the remaining two arc sodium, and two of R¹ to R⁴ are hydrogen and the remaining two are ammonium.

Among the bisphosphonic acid represented by Formula (I), more preferred are those wherein X represents C₁-C₈ linear or branched alkyl; Y represents C₁-C₈ linear or branched alkyl, trifluoromethyl, C₂-C₈ linear or branched alkenyl or C₃-C₈ cycloalkyl, C₁-C₈ alkoxy or halogen (excluding chlorine substituting atp-position); m and n represent 0 or 1; · · · represents single bond; A represents -S-(CH₂)_{c}-(wherein c represents an integer of 0, 1, 2 or 3); B represents hydrogen or C₁-C₈ linear or branched alkyl; and R¹, R², R³ and R⁴, the same or different, represent hydrogen, C₁-C₈ linear or branched alkyl, or pharmaceutically acceptable cation. More preferably, the compound is (4-methylthiophenyl)thiomethane-1,1-bisphosphonic acid or a salt thereof.

The methane bisphosphonic acid derivatives represented by Formula (I) *per se* are well-known in the art, and may be produced by well-known production processes. For example, they may be produced by the processes disclosed in Japanese Patent Publication (Kokoku) No. 8-26048 and U.S. Patent No. 5,527,940.

In the present invention, the bisphosphonic acid derivatives and the salts thereof may be used individually or two or more of these may be used in combination. The content thereof is preferably about 0.0001 to 1% by weight (1 µg/g to 10 mg/g) based on the entire composition.

The base used in the present invention is one which undergoes liquid-gel phase transition upon contact with physiological body fluid. The term "physiological body fluid" is the generic term of all the fluids which fill vessels or spaces between tissues or cells, and is a fluid which causes the base or the composition containing the base to gel, which base undergoes liquid-gel phase transition in the periodontal pocket, upon contact with the base or the composition. An example of the physiological body fluid in periodontal pocket is saliva. As the base which undergoes the phase transition upon contact with such a physiological body fluid, macromolecular substances which undergo the phase transition upon contact with the physiological body fluid are preferred. Examples thereof include gellan gum, sodium alginate, carageenan, guar gum, pectin, sodium polyacrylate, curdlan and the like. More preferred macromolecular substances are polysaccharides, especially gellan gum and carageenan.

Other preferred examples of the base are macromolecular substances which is made to gel by 1. the heat in the periodontal pocket, 2. the ion strength and/or 3. pH. Examples of the macromolecular substances which is made to undergo liquid-gel phase transition by 1. heat include polyoxyethylene-polyoxypropylene glycols which gel at the body temperature or the vicinity thereof such as Pluronic (registered trademark) and Poloxamer (registered trademark), and methylcellulose, hydraxycellulose and the like. As the macromolecular substances which is made to undergo liquid-gel phase transition by ion strength, the macromolecular substances which undergo liquid-gel phase transition upon contact with the physiological body fluid as mentioned above arc preferred, and examples thereof include gellan gum, sodium alginate, carageenan, guar gum, pectin, sodium polyacrylate, curdlan and the like.

The average molecular weight of the above-described macromolecular substances used as the base in the composition of the present invention is not restricted, and is usually about 10,000 to 10,000,000, preferably about 100,000 to 1,000,000.

Since the various macromolecular substances mentioned above used as the base in the composition of the present invention are commercially available as food materials, commercial products may be used.

The above-described composition is administered to periodontal pockets in the ungelled liquid form, and the composition gels at the administered site. In particular, when the composition is locally administered to a periodontal pocket for treating a periodontal disease, the administration of the composition to the periodontal pocket is simple, and the composition is in the ungelled liquid form having a fluidity or viscosity which enables administration of the composition to a deep portion of the periodontal pocket. After the administration, the composition gels upon contact with saliva and/or exudate from the periodontal diseased site, and the bisphosphonic acid derivative or the salt thereof contained therein is sustainedly released. More particularly, in cases where the base contained in the composition is gellan gum and/or carageenan, which are particularly preferred examples, by administering the composition in the form of a fluid aqueous solution containing the bisphosphonic acid derivative or the salt thereof to the periodontal pocket, the aqueous solution can reach the deep portion of the periodontal pocket, and gels *in situ* due to the calcium ion in the saliva and/or exudate from the periodontal diseased site. As a result, the bisphosphonic acid derivative or the salt thereof is sustainedly released without forming an insoluble salt. The term "fluid" herein does not define a concrete viscosity, but means that the composition is mobile without fixing its shape, that is, that the composition is not solid.

By preliminarily blending a compound which releases an ion such as calcium ion as an ion source in the composition, the gelling of the base which undergoes liquid-gel phase transition by ion strength may be enhanced. However, if an excess amount of such a compound is added, the fluidity of the composition may be degraded. Therefore, it is necessary not to add the compound at all, or to add the compound in a small amount with which gelation before the administration does not proceed.

The above-described bases which undergo liquid-gel phase transition may be used individually, or two or more of the bases may be used in combination. The bases which undergo liquid-gel phase transition by heat may be combined, the bases which undergo liquid-gel phase transition by ion strength may be combined, and the bases of these two types may also be combined. The total amount of the bases is preferably about 0.1 to 20% by weight based on the entire composition.

The bisphosphonic acid derivative or the salt thereof may exist in the pharmaceutical composition in dissolved state, suspended state, and/or in the state immobilized on particulate carriers. Examples of the particulate carriers include (1) microcapsules and nanocapsules wherein the bisphosphonic acid derivative or its salt is encapsulated in naturally occurring or synthetic macromolecules such as ethylcellulose, polylactic acid, polyglycolic acid, polylactic acid-polyglycolic acid copolymer, gelatin, polyacrylamide and the like; (2) small spheres (microspheres and nanospheres) made of albumin, starch, gelatin or polylactic acid; (3) emulsion and lipid microsphcres; (4) liposomes made of lipid bilayers; (5) microcarriers and nanocarriers made of substances having ion-exchange capacity, such as ion-exchange resins. As the base of these bisphosphonic acid derivatives, known pharmaceutically acceptable bases may be employed without restriction, and the particulate carriers may be prepared by the conventional formulation techniques. By incorporating the bisphosphonic acid derivative or its salt in the pharmaceutical composition in the immobilized form on particulate carriers, higher degree of sustainment of the release of the bisphosphonic acid derivative or its salt from the pharmaceutical composition may be attained.

The method for administering the composition to the above-described site is not restricted as long as the composition may be administered to the site where the bone resorption-inhibiting action of the bisphosphonic acid derivative or its salt is effectively exhibited, and a preferred administration method is the local administration by injection. Particularly, in cases where the pharmaceutical composition according to the present invention is administered to a periodontal pocket, it is preferred to administer the composition with the well-known injector having a hooked convergent nozzle as described in Japanese Laid-open Utility Model Application (Kokai) No. 1-62845, Japanese Laid-open Patent Application (Kokai) No. 4-117959, Japanese Utility Model No. 3035448 and Japanese Laid-open Patent Application (Kokai) No. 2000-107298.

The dose of the pharmaceutical composition according to the present invention is not restricted as long as the bone resorption-inhibiting action of the bisphosphonic acid derivative or its salt is effectively exhibited, and the dose of the composition may be administered to the above-described administration site. Usually, the composition having a volume of about 5% to 100% of the inner volume of the periodontal pocket is administered. Therefore, the dose in terms of the amount of the bisphosphonic acid derivative or its salt depends on the size of the periodontal pocket, and in many cases, about 0.1 ng to 1 mg/pocket.

To the pharmaceutical composition according to the present invention, other components such as known vehicles, binders, coloring agents, correctives, flavors, surfactants, sweeteners, antiseptics and the like may appropriately be added depending on the purpose and the type of the composition. The pharmaceutical composition according to the present invention may be prepared by the usual formulation techniques. Preferred formulation examples are described in the Examples below.

### Examples

The present invention will now be described by way of examples and experiments. All the values indicating the added amount are "% by weight" unless otherwise specified. The "bisphosphonic acid compound" means (4-methylthiophenyl)thiomethane-1,1-bisphosphonic acid disodium (described in Japanese Patent Publication (Kokoku) No. 8-26048 and U.S. Patent No. 5,527,940) unless otherwise specified. The artificial saliva used as the test fluid was the aqueous solution having the following composition, which was prepared based on the teaching by Biomaterials 20, 55-60 (1999).

### Composition of Artificial Saliva

| | |
|---|---|
| Potassium dihydrogen phosphate: | 2.5 mM/L |
| Disodium hydrogen phosphate: | 2.4 mM/L |
| Potassium hydrogen carbonate: | 15.0 mM/L |
| Sodium chloride: | 10.0 mM/L |
| Magnesium chloride: | 1.5 mM/L |
| Calcium chloride: | 1.5 mM/L |
| Citric Acid | 0.15 mM/L |

The pH was adjusted to 6.7 by sodium hydroxide or hydrochloric acid.

### Example 1

**Table 1**

| Component | (Manufacturer, Trademark) | Added Amount (%) |
|---|---|---|
| Bisphosphonic acid compound | (Toray) | 0.20 |
| Gellan gum | (San-Ei Gen F.F.I, GELRITE) | 1.00 |
| Distilled Water | (Otsuka Pharmaceutical, Distilled water for injection) | 98.80 |
| Total | | 100.00 |

Bisphosphonic acid compound was dissolved in distilled water, and this aqueous solution was heated to a temperature not lower than 90°C. Gellan gum was added thereto, well dispersed and dissolved by continuously stirring the solution to obtain the composition in ungelled liquid form shown in Table 1.

### Example 2

**Table 2**

| Component | (Manufacturer, Trademark) | Added Amount (%) |
|---|---|---|
| Bisphosphonic acid compound | (Toray) | 0.20 |
| Carageenan | (Ina Shokuhin Kogyo, Carageenan PA-5) | 1.75 |
| Distilled Water | (Otsuka Pharmaceutical, Distilled water for injection) | 98.05 |
| Total | | 100.00 |

Bisphosphonic acid compound was dissolved in distilled water, and this aqueous solution was heated to a temperature not lower than 80°C. Carageenan was added thereto, well dispersed and dissolved by continuously stirring the solution to obtain the composition in ungelled liquid form shown in Table 2.

### Example 3

**Table 3**

| Component | (Manufacturer, Trademark) | Added Amount (%) |
|---|---|---|
| Bisphosphonic acid compound | (Toray) | 0.20 |
| Carageenan | (Ina Shokuhin Kogyo, Carageenan PA-5) | 0.75 |
| Gcllan gum | (San-Ei Gen F.F.I, GELRITE) | 0.75 |
| Distilled Water | (Otsuka Pharmaceutical, Distilled water for injection) | 98.30 |
| Total | | 100.00 |

Bisphosphonic acid compound was dissolved in distilled water, and this aqueous solution was heated to a temperature not lower than 80°C. Carageenan was added thereto, well dispersed and dissolved by continuously stirring the solution. Then the resulting solution was heated to a temperature not lower than 90°C, and gellan gum was added thereto and well dispersed to obtain the composition in ungelled liquid form shown in Table 3.

The release property of the bisphosphonic compound from the test substance was tested by the following experiment The experiment apparatus and conditions were in accordance with the Japanese Pharmacopoeia, Dissolution Test, Second Method (amount of test fluid: 500 mL, test fluid temperature: 37°C, paddle revolution: 50 rpm).

### Experiment Example (Elution Test)

On the bottom of an ointment cell (cylinder having bottom, made of acrylic resin, inner diameter 31 mm, height 5 mm), 3.8 g of the test substance is placed. A screen (stainless steel wire gauze, outer diameter 35 mm, mesh 500 µm) was placed thereon, and ointment cell cover (cylinder with diameter of 31 mm, made of acrylic resin, having an opened top plate) is put thereon so as to prevent the screen from being displaced, thereby fixing the screen gauze.

Distilled water as the test fluid was placed in a beaker, and the above-described ointment cell containing the sampled compositions prepared in Example 1 was immersed therein, followed by measuring the percentage of the bisphosphonic acid compound released into the test fluid with time.

Similarly, the artificial saliva as the test fluid was placed in beakers, and the above-described ointment cells containing the sampled composition prepared in Example 1, Example 2 and Example 3, respectively, were separately immersed in the beakers, followed by measuring the percentage of the bisphosphonic acid compound eluted into the test fluid with time. The results are shown in Fig. 1.

As can be seen from the results, although the bisphosphonic acid compound contained in the composition according to the present invention was quickly released into distilled water, the bisphosphonic acid compound was sustainedly released into the artificial saliva without forming an insoluble salt with calcium ion.

### Industrial Availability

Since the composition according to the present invention enables sustained-release of bisphosphonic acid compounds which were hitherto thought difficult to be administered to periodontal pockets, and enables to selectively deliver the bisphosphonic compounds to alveolar bone, the composition is useful as a alveolar bone resorption-inhibiting agent and as a pharmaceutical composition for therapies of periodontal diseases.

## Claims

1. A pharmaceutical composition for being administered to periodontal pockets, comprising a bisphosphonic acid derivative or a salt thereof, and a base which undergoes liquid-gel phase transition upon contact with physiological body fluid in the periodontal pocket.

2. The pharmaceutical composition for being administered to periodontal pockets according to claim 1, wherein said bisphosphonic acid derivative or the salt thereof is a methane bisphosphonic acid derivative represented by Formula (I): 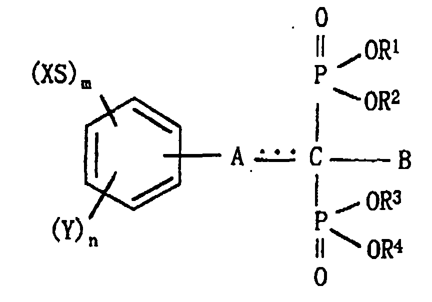 [wherein X represents C₁-C₈ linear or branched alkyl or cycloalkyl (in case of cycloalkyl, the number of carbon atoms is 3 to 8) which is not substituted or which has (a) substituent(s) having nitrogen, oxygen and/or silicon atom(s), phenyl or naphthyl (the phenyl or naphthyl may be substituted by C₁-C₈ linear or branched alkyl or cycloalkyl (in case of cycloalkyl, the number of carbon atoms is 3 to 8), C₁-C₈ linear or branched alkoxy, halogen and/or hydroxy); Y represents C₁-C₈ linear or branched alkyl, trifluoromethyl, C₂-C₈ linear or branched alkenyl, C₃-C₈ cycloalkyl, C₁-C₈ alkoxy or halogen (excluding chlorine substituting at *p*- position); m and n independently represent 0, 1, 2 or 3; · · · represents double bond or single bond; A represents -(D)_{b}-(CH₂)_{c}- (wherein D represents sulfur, oxygen, NR⁵ (wherein R⁵ represents hydrogen or C₁-C₈ linear or branched alkyl or cycloalkyl (in case of cycloalkyl, the number of carbon atoms is 3 to 8), D binding directly to the methane bisphosphonic acid, c represents an integer of 0 to 3, b represents 0 or 1), or -(CH=CH)_{d}-CH= (wherein d represents 0 or 1, and when A is -(CH=CH)_{d}-CH=, B does not exist); B represents hydrogen, C₁-C₈ linear or branched alkyl or cycloalkyl (in case of cycloalkyl, the number of carbon atoms is 3 to 8), hydroxy or trialkylsiloxy (each of the alkyl groups therein is C₁-C₈ linear or branched alkyl or cycloalkyl (in case of cycloalkyl, the number of carbon atoms is 3 to 8); R¹, R², R³ and R⁴, the same or different, represent hydrogen, C₁-C₈ linear or branched alkyl or cycloalkyl (in case of cycloalkyl, the number of carbon atoms is 3 to 8), or a pharmaceutically acceptable cation]
or a hydrate thereof.

3. The pharmaceutical composition for being administered to periodontal pockets according to claim 1 or 2, wherein X in said Formula (I) represents C₁-C₈ linear or branched alkyl; Y represents the same meanings as described above; m and n independently represent 0 or 1; · · · represents single bond; A represents -S-(CH₂)_{c}- (wherein c represents an integer of 0 to 3); B represents hydrogen or C₁-C₈ linear or branched alkyl; and R¹, R², R³ and R⁴ represent the same meanings as in claim 2.

4. The pharmaceutical composition for being administered to periodontal pockets according to any one of claims 1 to 3, wherein said base is at least one polysaccharide.

5. The pharmaceutical composition for being administered to periodontal pockets according to claim 4, wherein said polysaccharide is gellan gum and/or carageenan.

6. The pharmaceutical composition for being administered to periodontal pockets according to any one of claims 1 to 5, which is for therapy of a periodontal disease.

7. A method for treating periodontal pockets, comprising administering an effective amount of said composition according to any one of claims 1 to 5 to a periodontal pocket

8. A therapeutic method for a periodontal disease, comprising administering an effective amount of said composition according to any one of claims 1 to 5 to a periodontal pocket.

9. Use of said composition according to any one of claims 1 to 5, for the production of a pharmaceutical composition for being administered to periodontal pockets.

10. Use of said composition according to any one of claims 1 to 5, for the production of a pharmaceutical composition for therapy of a periodontal disease.
